# EUROPÄISCHE PATENTSCHRIFT

(11) **EP 2 131 799 B1**
(45) Veröffentlichungstag und Bekanntmachung des Hinweises auf die Patenterteilung: **21.08.2013**
(21) Anmeldenummer: 08715492.8
(22) Anmeldetag: 13.02.2008
(51) Int. Cl.: A61F 5/00

(54) **STÜTZFEDER**
RESILIENT SUPPORT
RESSORT D'APPUI

(30) Priorität: 23.02.2007 DE 102007008933; 22.03.2007 DE 102007013823; 26.10.2007 DE 102007051652
(43) Veröffentlichungstag der Anmeldung: 16.12.2009
(73) Patentinhaber: Gottinger Handelshaus GbR, 85604 Zorneding (DE)
(72) Erfinder: GÜNTHER, Norbert, G., 85599 Parsdorf (DE)
(74) Vertreter: Winter, Brandl, Fürniss, Hübner, Röss, Kaiser, Polte - Partnerschaft
(86) Internationale Anmeldenummer: PCT/DE2008/000272
(87) Internationale Veröffentlichungsnummer: WO 2008/101472

(56) Entgegenhaltungen:
- EP-A- 1 714 623
- WO-A-2004/069087
- DE-B- 1 140 312
- US-A- 1 354 427
- US-A- 2 444 839

## Beschreibung

Die Erfindung betrifft eine Stützfeder nach dem Oberbegriff des Patentanspruchs 1.

Derartige Stützfedern werden beispielsweise bei Unterschenkelorthesen für Patienten mit tiefen Lähmungen, bei Muskelerkrankungen, infantilen Zelebralparesen, pathologischen Erkrankungen, neurologischen Veränderungen oder auch bei gesunden Menschen eingesetzt, um die Funktion der Plantarflektoren zu unterstützen. Durch die Unterschenkelorthese wird der Fuß in Bezug zum Unterschenkel gestützt, wobei gleichzeitig durch die Stützfeder während der Auftritt- und Standphase Energie aufgenommen und diese bei der Abstoßphase wieder zurückgegeben wird.

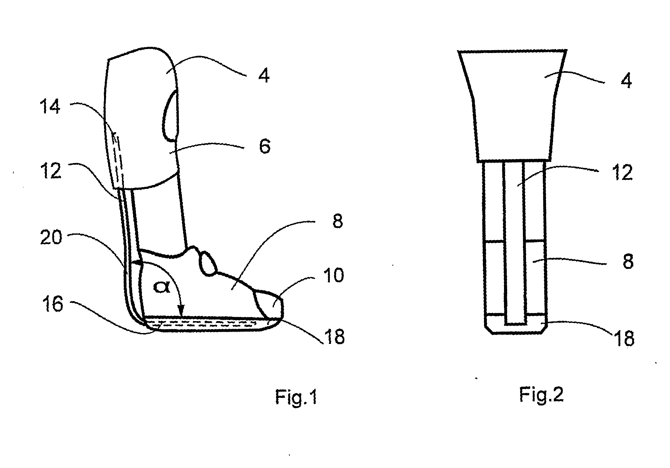

Eine bekannte Stützfeder sowie eine Unterschenkelorthese zeigen die Figuren 1 und 2. Die Figuren gehen zurück auf die Unterschenkelorthese SPRING der Anmelderin und sind dem Katalog Medizinisches Verordnungsprogramm, Fa. Gottinger GmbH, 85604 Zornerding, zu entnehmen. Die Unterschenkelorthese 2 hat eine Unterschenkelmanschette 4 zum Umgreifen eines Unterschenkels 6 und eine Fußmanschette 8 zum Festhalten eines Fußes 10. Die beiden Manschetten 4, 6 sind über eine Stützfeder 12 mit einem unterschenkelseitigen Endabschnitt 14 und einen fußseitigen Endabschnitt 16 gelenkig miteinander verbunden, wobei der unterschenkelseitige Endabschnitt 14 in der Unterschenkelmanschette 4 und der fußseitige Endabschnitt 16 in einer den Fuß 10 tragenden Sohle 18 aufgenommen ist. Zur Unterstützung der Federwirkung ist ein Fersenteil 20 der Stützfeder 12 gegenläufig gekrümmt.

Nachteilig an dieser bekannten Lösung ist, dass die Federrate der Stützfeder so ausgelegt ist, dass eine bestmögliche Unterstützung in der Abstoßphase beim Gehen erfolgt. Aufgrund des Bewegungsablaufs beim Gehen muss die Stützfeder jedoch in der Abstoßphase eine größere Stützkraft als in der Auftretphase entwickeln, so dass bei Verwendung der bekannten Lösung kein "weiches" Auftreten ermöglicht ist und somit ein störendes "Schieben der Stützfeder in die Knie" bzw. ein kräftiger Rückschlag des Fußes in Richtung des Knies erfolgt.

Des Weiteren ist nachteilig, dass ein Patient zum Ausführen einer selbständigen Plantarflexion des Fußes mit Muskelkraft, wie zum Beispiel zum Betätigen der Fußpedale beim Führen eines Kraftfahrzeugs notwendig, aufgrund der hohen Federrate stets eine hohe Gegenkraft aufbringen muss, um gegen die Federkraft der Stützfeder gegenanzuwirken.

Aufgabe der vorliegenden Erfindung ist es, eine Stützfeder zu schaffen, die die vorgenannten Nachteile beseitigt und kostengünstig herzustellen ist.

Diese Aufgabe wird gelöst durch eine Stützfeder mit den Merkmalen nach dem Patentanspruch 1

Die erfindungsgemäße Stützfeder, für eine Unterschenkelorthese, zum gelenkigen Verbinden einer Unterschenkelmanschette mit einer Fußmanschette hat einen unterschenkelseitigen und einen fußseitigen Endabschnitt, die durch ein Fersenteil miteinander verbunden sind, wobei die Stützfeder in Abhängigkeit von der Bewegungsrichtung (Plantar- /Dorsalflexion) des Fußes unterschiedliche Federraten aufweist, da im Fersenbereich ein Schlitz ausgebildet ist. Dies hat den Vorteil, dass bei einer Plantarflexion, die beim Auftreten erfolgt, die Stützfeder weicher ist, somit der Fuß weicher auftritt und bei einer Dorsalflexion, beim Abstoßen des Fußes, härter ist und das Abstoßen unterstützt.

Vorzugsweise ist der Schlitz zum unterschenkelseitigen und zum fußseitigen Endabschnitt hin geschlossen.

Bei einem Ausführungsbeispiel wird der Schlitz an seinen unterschenkel- und fußseitigen Schlitzenden verwoben, beispielsweise mit einem Kevlarfaden, wodurch diese zuverlässig verschlossen und verstärkt werden und der Schlitz sich im Einsatz und bei der Benutzung der Stützfeder nicht undefiniert vergrößern kann.

Der wesentliche Vorteil der erfindungsgemäßen Stützfeder besteht darin, das diese übereinanderliegende Schichten hat, welche durch den Schlitz separiert sind, wobei diese unterschiedliche Dicken haben können, von welcher die Federsteifigkeit der Stützfeder abhängt. Zur Vereinfachung der Herstellung befindet sich im Schlitz bevorzugt eine Trennfolie.

Zur Unterstützung der Federwirkung der Stützfeder kann das Fersenteil in Bezug zum unterschenkelseitigen und zum fußseitigen Endabschnitt gegenläufig gekrümmt sein, sowie vorzugsweise aus einem faserverstärkten, beispielsweise kohlefaserverstärkten, Kunststoff hergestellt werden.

In dem Schlitz kann bei einem bevorzugten Ausführungsbeispiel ein Einlageelement eingebracht sein, womit die bei einer Dorsalextension aufeinandertreffenden Schichten gedämpft werden können.

Zur Verbesserung der Dämpfung kann das Material des Einlageelements beispielsweise ein Kunststoff, insbesondere ein Elastomer, sein.

Vorzugsweise ist das Einlageelement an einer Innenfläche mit der Stützfeder stoffschlüssig verbunden, um eine Lageänderung des Einlageelements im Einsatz der Stützfeder zu vermeiden. Die Außenfläche des Einlageelement ist dabei frei, womit sich ein Spalt zwischen der Stützfeder und dem Einlageelement bilden kann.

Um bei beispielsweise einem Materialversagen der Stützfeder eine Verletzung des Stützfederpatienten zu vermeiden, kann bei dieser ein Schutzelement vorgesehen sein.

Das Schutzelement ist vorteilhafterweise mit der Oberfläche der Stützfeder stoffschlüssig verbunden.

Beispielsweise ist das Schutzelement an der Innenseite und Außenseite der Stützfeder im Bereich des unterschenkelseitigen Endabschnitt und dem Fersenteil angeordnet, da in diesem Bereich hohe Belastungen der Stützfeder vorherrschen können.

Sonstige vorteilhafte Ausführungsformen sind Gegenstand weiterer Unteransprüche.

### Ausführungsbeispiele

Im Folgenden werden bevorzugte Ausführungsbeispiele der Erfindung anhand schematischer Zeichnungen näher erläutert: Es zeigen:
Fig. 1 eine Seitenansicht einer bekannten Unterschenkelorthese mit einer bekannten Stützfeder;
Fig. 2 eine Rückansicht der bekannten Unterschenkelorthese aus Fig. 1;
Fig. 3 eine Seitenansicht einer erfindungsgemäßen Stützfeder gemäß einem ersten Ausführungsbeispiel;
Fig. 4 eine Seitenansicht eines Schlitzebereichs der Stützfeder gemäß dem ersten Ausführungsbeispiel;
Fig. 5 eine Seitenansicht des Schlitzebereichs der Stützfeder gemäß dem ersten Ausführungsbeispiel;
Fig. 6 eine Seitenansicht des Schlitzebereichs der Stützfeder gemäß einem zweiten Ausführungsbeispiels; und
Fig. 7 eine Vorderansicht der Stützfeder mit einer Ausbruchsdarstellung des Einlagenbereichs gemäß dem zweiten Ausführungsbeispiel.

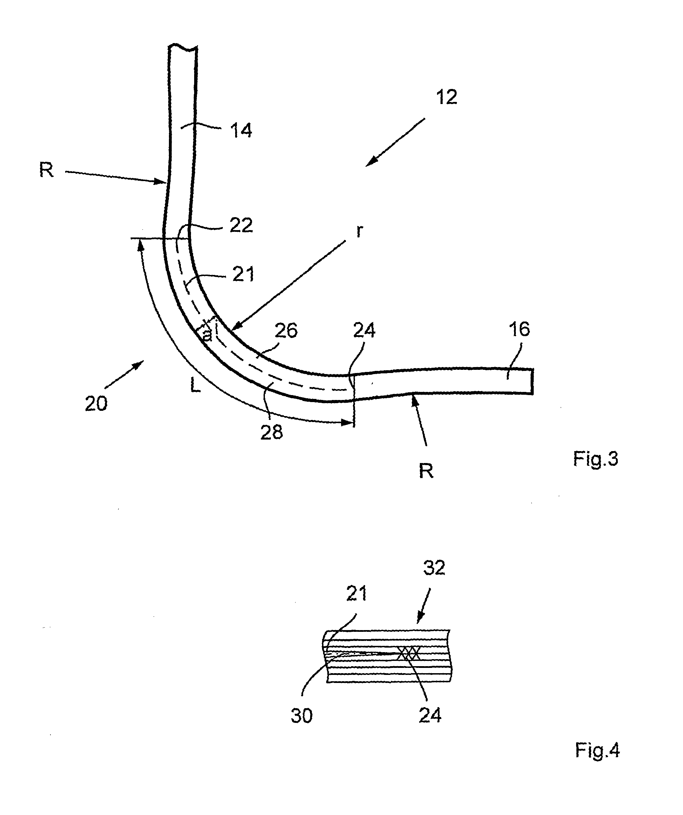

Fig. 3 zeigt eine bevorzugte Ausführungsform einer erfindungsgemäßen Stützfeder 12. Die Stützfeder 12 hat einen etwa L-förmigen Aufbau mit einem unterschenkelseitigen Endabschnitt 14 und einem fußseitigen Endabschnitt 16, die von einem gegenläufig gekrümmten Fersenteil 20 miteinander verbunden sind. Die Stützfeder 12 ist als Blattfeder ausgebildet, wobei als Material vorzugsweise ein faserverstärkter Kunststoff, beispielsweise ein glasfaserverstärktem oder kohlefaserverstärkter Kunststoff oder ein Verbundwerkstoff, verwendet wird. Dieses Material zeichnet sich, bei minimalen Gewicht, durch eine hervorragende Biegesteifigkeit und hoher Dauerfestigkeit aus. Prinzipiell lassen sich jedoch auch andere geeignete Materialien einsetzen, die jedoch stets im Hinblick auf minimales Gewicht und maximale Dauerfestigkeit auszuwählen sind.

Im Fersenbereich der Stützfeder 12 ist ein Schlitz 20 ausgebildet, der etwa mittig verläuft, sich entlang des Fersenteils 20, über den gesamten gekrümmten Fersenbereich erstreckt und jeweils in Richtung zu den Endabschnitten 14, 16 hin endet.

Fig. 4 zeigt eine Detaildarstellung eines Schlitzendes 24. Bei der Herstellung werden die nicht vorgetränkten Gewebelagen 32 zugeschnitten und aufeinandergelegt, wobei im Schlitzbereich eine Trennfolie 30 eingelegt wird, welche sich über die gesamte Länge L des Schlitzes 20 bis zu den Enden 22, 24 erstreckt. Die an die Trennfolie 30 angrenzenden Bereiche werden mit einem Kevlarfaden vernäht, so dass die Folie fixiert und die Schlitzlänge definiert ist. Danach wird der Mehrschichtaufbau in einem Werkzeug mit Matrixharz getränkt und ausgehärtet.
Eine andere Möglichkeit der Herstellung ist, mit Matrixharz vorgetränkte Gewebelagen 32 zu verwenden. Die Herstellungsschritte der Stützfeder 12 sind hierbei die gleichen, wobei der Mehrschichtaufbau nicht mehr mit Matrixharz getränkt wird, sondern nur noch gehärtet wird.

Die Trennfolie 30 bleibt nach der Herstellung im Schlitz, wodurch die Reibung im Einsatz der Stützfeder 12 minimiert wird und die Dauerfestigkeit erhöht.

Um unterschiedliche Federkräfte der beiden Schichten 26, 28 zu realisieren, kann es vorteilhaft sein, wenn diese in mittiger Richtung betrachtet unterschiedliche Dicken a, i aufweisen. Dabei ist die Gesamtdicke g der Stützfeder 12 über ihrer Gesamtlänge im Wesentlichen konstant, so dass in jedem Körperabschnitt 14, 16, 20 gilt g=a+i.

Die Krümmung des Fersenteils 20 wird durch zwei Radien R und r ausgebildet. Der Radius r und der sich bis zur Unterschenkelmanschette 4 (s. Figuren 1 und 2) erstreckende Teil der Stützfeder 12 bestimmen im Wesentlichen die Beweglichkeit der Stützfeder 12 bei einer Plantarflexion, während der Radius R die Beweglichkeit der Stützfeder 12 bei einer Dorsalextension (d.h. die Drehung des Fußes um das obere Sprunggelenk, so dass sich der Fußrücken der Unterschenkelvorderseite annähert), überwiegend bestimmt. In der Regel wird man die Stützfeder 12 so auslegen, dass weniger Unterstützung einer Dorsalextension erfolgt, sondern vorwiegend eine Unterstützung einer Plantarflexion. Die Beweglichkeit wird dabei in den Bereich in der Nähe des oberen Sprunggelenks gelegt.

Die Unterschenkelmanschette 4 und die Fußmanschette 8 unterscheiden sich nicht wesentlich vom Stand der Technik gemäß den Fig. 1 und Fig. 2, so dass auf eine nochmalige Erläuterung verzichtet wird.

Die Funktion und Wirkungsweise der erfindungsgemäßen Stützfeder 12 in Verbindung mit der Unterschenkelorthese 2 wird im Folgenden erläutert:
Die Unterschenkelorthese 2 umgreift mit ihrer Unterschenkelmanschette 4 und ihrer Fußmanschette 8 den Unterschenkel 6 und den Fuß 10 eines Patienten. Über die Stützfeder 12 sind die Manschetten 4, 8 gelenkig miteinander verbunden, so dass der Patient mit seinem Fuß 10 um das obere Sprunggelenk Dorsalextensionen und Plantarflexionen ausführen kann bzw. diese unterstützt werden.
Dabei ist die Stützfeder 12 an ihren Endabschnitten 14, 16 fest in die Manschetten 4, 8 integriert.

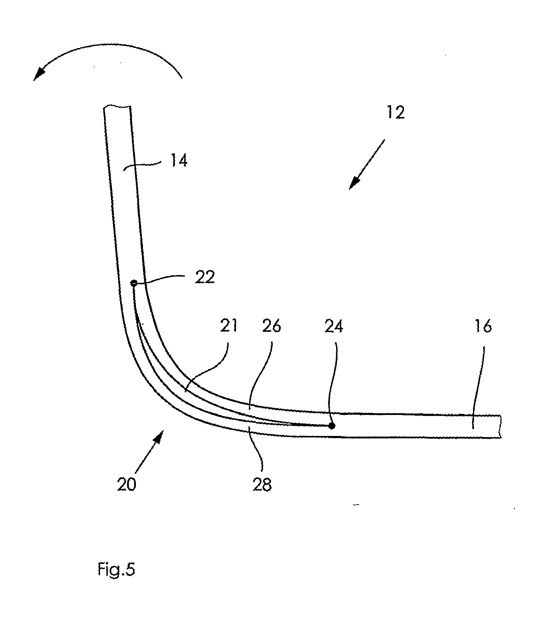

Ab einem bestimmten Größe des Winkels (α) der Plantarflexion beult die Außenschicht 28 aus, wie in Fig. 5 gezeigt, dadurch wird der Abstand der Schichten 26, 28 zueinander größer und somit vergrößert sich die Höhe des Schlitzes 21. Durch das Ausbiegen/ Ausbeulen der Außenschicht 28, werden die Federspannungen auf die Innenschicht 26 verlagert und diese wird dadurch stärker auf Zug belastet. Die Außenschicht 28 wird gleichzeitig entspannt oder auf eine Biegespannung belastet, die wesentlich geringer als die Zugbelastung der Innenschicht ist. Je größer der Winkel (α) der Plantarflexion, desto kleiner werden die Spannungen in der Außenschicht 28 und desto größer in der Innenschicht (26). Der tragende Querschnitt der Innenschicht bestimmt dann die Steifigkeit der Stützfeder 12, welche somit sinkt. Wird der Winkel der Plantarflexion kleiner, so geht die Ausbeulung der Außenschicht 28 zurück, bis die beider Schichten 26, 28 wieder aufeinanderliegen. Ab diesem Winkel (α) hat die Stützfeder 12 konstant die höchste Steifigkeit, da beide Schichten die Federspannungen aufnehmen und gespannt sind. Die Federrate ist dann durch den Querschnitt beider Schichten bestimmt.
Durch diese Abhängigkeit der Federsteifigkeit von dem Winkel (α) der Plantarflexion weist die Stützfeder 12 eine progressive Federkennlinie auf, deren Federsteifigkeit mit steigender Plantarflexion abnimmt.

Die Funktion einer Stützfeder 12 bei den einzelnen Gangphasen wird in DE 103 05 131 B4 beschrieben, so dass diesbezüglich der Einfachheit halber auf diese Ausführungen verwiesen wird.

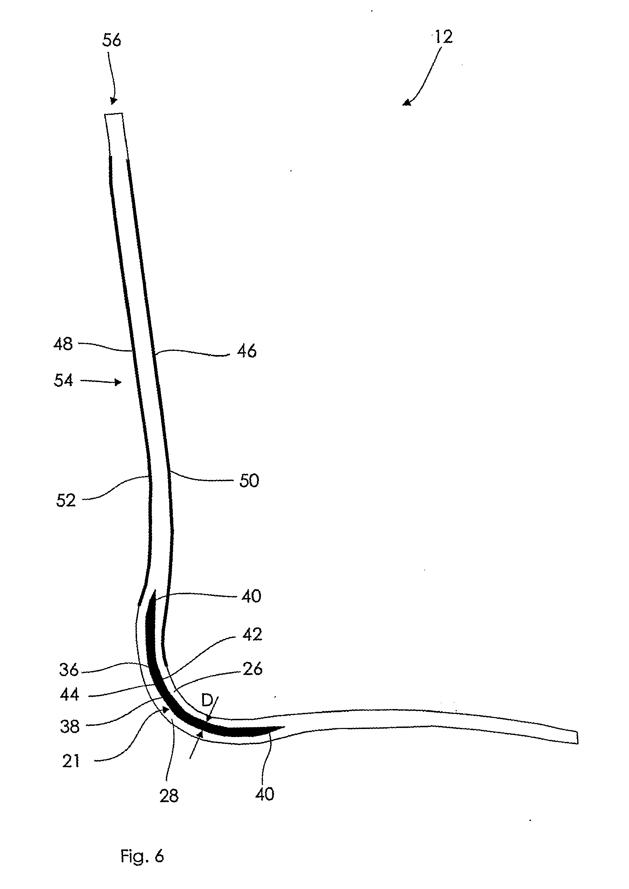

Figur 6 zeigt eine Seitenansicht der Stützfeder 12 gemäß einem zweiten Ausführungsbeispiel. Diese weist ein elastisches Einlageelement 36 aus einem Elastomer auf, das in den Schlitz 21 eingebracht ist und diesen im Wesentlichen ausfüllt. Das Einlageelement 36 hat dabei einen Mittelbereich 38 mit in etwa konstanter Dicke D, beispielsweise zwischen 1 und 3 mm, und zwei spitz zulaufende Endbereiche 40. An einer Innenfläche 42 ist das Einlageelement 36 mit der Innenschicht 26 der Stützfeder 12 stoffschlüssig verbunden, dagegen ist eine Außenfläche 44 von der Außenschicht 28 getrennt und nicht mit dieser verbunden.

Im Einsatz der Stützfeder 12 bewegen sich die Innenschicht 26 und Außenschicht 28, siehe auch Fig. 5, bei einer Plantarflexion auseinander. Bei der darauf folgenden Dorsalextension treffen diese bei der vorbeschriebenen Stützfeder dann wieder aufeinander, was einen harten Stoß zur Folge hat, falls kein Einlageelement 36 in den Schlitz 21 eingebracht ist. Dieser Stoß führt beispielsweise zu Schäden im Knie eines Stützfederpatienten oder auch zu einer vorzeitigen Materialermüdung der Stützfeder 12. Zum Abdämpfen des Stoßes dient beim Ausführungsbeispiel gemäß der Figur 6 das elastische Einlageelement 36. Bei einer Plantarflexion bildet sich bei einer derartigen Stützfeder 12 ein Spalt zwischen dem Einlageelement 36 und der Außenschicht 28. Bei der folgenden Dorsalextension stößt die Außenschicht 28 dann auf das elastische Einlageelement 36, das eine progressive von der Shore-Härte abhängige Federsteifigkeit aufweist und den Stoß abdämpft, wodurch das Knie des Stützfederbenutzers geschont wird.

In der Figur 6 sind bei der Stützfeder 12 zwei Schutzelemente 46, 48 angeordnet, die bei einem Materialversagen der Stützfeder 12 ein aussplittern verhindern. Das Schutzelement 46 ist dabei an einer Innenfläche 50 und das Schutzelement 48 an einer Außenfläche 52 der Stützfeder 12 stoffschlüssig fixiert. Diese erstrecken sich in etwa entlang eines unterschenkelseitigen Stützfederabschnitt 54 der Stützfeder 12 und enden im Bereich des Fersenteils 20, wobei das innere Schutzelement 46 in etwa in der Mitte des Ferstenteils 20 und das äußere Schutzelement 48 im Übergangsbereich zwischen Stützfederabschnitt 54 und Fersenteil 20 endet. Die Schutzelemente 46, 48 sind von einem unterschenkelseitigen Stützfederende 56 der Stützfeder etwas beabstandet. Das Material der Schutzelemente 46, 48 ist äußerst strapazierfähig und beispielsweise ein elastischer Kunststoff, der auf die Stützfeder 12 aufvakuumiert wird.

Bei einem Bruch oder ähnlichen Schäden der Stützfeder 12 verhindern die Schutzelemente 46, 48 ein Aussplittern des Stützfedermaterials, das bei einem Stützfederr zu Verletzungen führen könnte.

Anstatt zwei Schutzelementen 46, 48 kann auch ein Element die Stützfeder 12 abschnittsweise oder im Ganzen ummanteln.

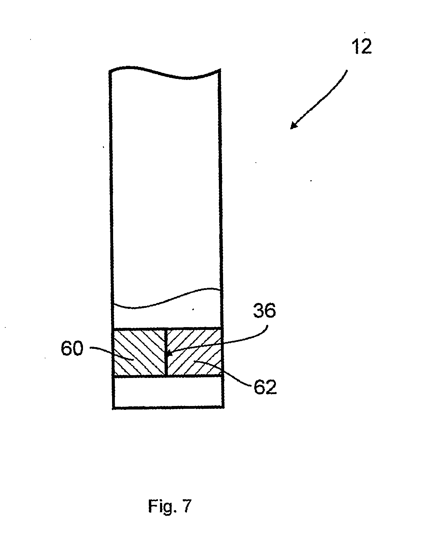

In der Figur 7 ist eine Vorderansicht der Stützfeder 12 gemäß dem zweiten Ausführungsbeispiel gezeigt, wobei der Bereich des Einlageelements 36 freigeschnitten ist. Die Shore-Härte des Einlageelements 36 beträgt beispielsweise 65. Es ist durchaus denkbar, dass dieses Abschnitte mit unterschiedlichen Shore-Härten aufweist. Beispielsweise hat das Einlageelement 36 in der Vorderansicht in Figur 7 zwei Einlagebereiche 60, 62 mit jeweils einer Breite, die die Hälfte der Gesamtbreite der Stützfeder 12 beträgt. Der in Figur 7 linke Einlagebereich 60 könnte dabei eine höhere Shore-Härte, wie der andere Einlagebereich 62 aufweisen, wodurch bei einer Dorsalextension der weichere Einlagebereich 62 leichter einfedern würde als der härtere. Dies hätte zur Folge, dass die Stützfeder 12 dabei tordiert wird und somit einen zusätzlichen Freiheitsgrad zur Anpassung an bestimmte Geheigenschaften eines Stützfederpatienten hätte.

Offenbart ist eine Stützfeder, welche im Fersenteil einen Schlitz aufweist.

### Bezugszeichenliste

- 2: Unterschenkelorthese
- 4: Unterschenkelmanschette
- 6: Unterschenkel
- 8: Fußmanschette
- 10: Fuß
- 12: Stützfeder
- 14: unterschenkelseitiger Endabschnitt
- 16: fußseitiger Endabschnitt
- 18: Sohle
- 20: Fersenteil
- 21: Schlitz
- 22: unterschenkelseitiges Schlitzende
- 24: fußseitiges Schlitzende
- 26: Innenschicht
- 28: Außenschicht
- 30: Trennfolie
- 32: Gewebelage
- 34: Kevlarfaden
- 36: Einlageelement
- 38: Mittelbereich
- 40: Endbereich
- 42: Innenfläche
- 44: Außenfläche
- 46: Schutzelement
- 48: Schutzelement
- 50: Innenfläche
- 52: Außenfläche
- 54: Stützfederabschnitt
- 56: Stützfederende
- 60: Einlagebereich
- 62: Einalgebereich

## Patentansprüche

1. Stützfeder, für eine Unterschenkelorthese, zum gelenkigen Verbinden einer Unterschenkelmanschette mit einer Fußmanschette, mit einem unterschenkelseitigen und einem fußseitigen Endabschnitt (14, 16), die über ein Fersenteil (20) miteinander verbunden sind, wobei die Stützfeder in Abhängigkeit vom Drehwinkel (α) des Fußes unterschiedliche Federraten aufweist, **dadurch gekennzeichnet, dass** im Fersenbereich wenigstens ein Schlitz (21) vorgesehen ist.

2. Stützfeder nach Patentanspruch 1, wobei der Schlitz (21) zum unterschenkelseitigen und zum fußseitigen Endabschnitt (14, 16) hin geschlossen ist.

3. Stützfeder nach Patentanspruch 2, wobei der Schlitz (21) ein unterschenkel- und ein fußseitiges Schlitzende (22, 24) hat, welche jeweils verwoben oder verklebt sind.

4. Stützfeder nach einem der vorhergehenden Ansprüche, wobei sich durch den Schlitz (21), zwei übereinanderliegende Schichten (26, 28) ergeben, welche gleiche oder unterschiedliche Dicken (a, i) aufweisen.

5. Stützfeder nach einem der vorhergehenden Ansprüche, wobei sich der Schlitz etwa in der neutralen Zone der Stützfeder befindet.

6. Stützfeder nach einem der vorhergehenden Patentansprüche, wobei das Fersenteil (20) gegenläufig gekrümmt zum dem fußseitigen und zum unterschenkelseitigen Endabschnitt (14, 16) ist.

7. Stützfeder nach Patentanspruch 3 bis 6, wobei die Schlitzenden (22, 24) mit einem Kevlarfaden (34) verwoben sind.

8. Stützfeder nach einem der vorhergehenden Patentansprüche, wobei sich im Schlitz (21) eine Trennfolie befindet, welche die Schichten (26, 28) separiert.

9. Stützfeder nach einem der vorhergehenden Patentansprüche, wobei die Stützfeder (12) eine Blattfeder aus faserverstärktem Kunststoff ist.

10. Stützfeder nach Patentanspruch 9, wobei die Blattfeder entweder aus einem kohlefaserverstärktem Kunststoff, glasfaserverstärktem Kunststoff oder einem anderen Verbundwerkstoff ist.

11. Stützfeder nach einem der vorhergehenden Patentansprüche, wobei in dem Schlitz (21) ein Einlageelement (36) eingebracht ist.

12. Stützfeder nach Anspruch 11, wobei das Material des Einlageelements (36) ein Kunststoff, insbesondere einem Elastomer, ist.

13. Stützfeder nach Anspruch 11 oder 12, wobei das Einlageelement (36) an einer Innenfläche (42) mit der Stützfeder (12) stoffschlüssig verbunden ist und an einer Außenfläche (44) frei ist.

14. Stützfeder nach Anspruch 11 oder 12, wobei das Einlageelement (36) Einlageabschnitte mit unterschiedlichen Shore-Härten hat.

15. Stützfeder nach einem der vorhergehenden Ansprüche, wobei an der Oberfläche der Stützfeder (12) wenigstens ein Schutzelement (46) angeordnet ist.

16. Stützfeder nach Anspruch 15, wobei das Schutzelement (46) mit der Oberfläche der Stützfeder (12) stoffschlüssig verbunden ist.

17. Stützfeder nach Anspruch 15 oder 16, wobei das Schutzelement (46) an der Innenfläche (50) und Außenfläche (52) der Stützfeder (12) im Bereich des unterschenkelseitigen Stützfederabschnitts (54) und dem Fersenteil (20) angeordnet ist.

## Claims

1. A resilient support for a below-knee orthotic device for an articulated connection of a below-knee cuff to a foot cuff, comprising a lower leg-side end portion and a foot-side end portion (14, 16) interconnected via a heel part (20), wherein the resilient support has different spring rates in response to the angle of rotation (α) of the foot, **characterized in that** in the heel area at least one slit (21) is provided.

2. A resilient support according to claim 1, wherein the slit (21) is closed toward the lower leg-side and the foot-side leg portions (14, 16).

3. A resilient support according to claim 2, wherein the slit (21) has a lower leg-side slit end and a foot-side slit end (22, 24) each of which is interwoven or bonded.

4. A resilient support according to any one of the preceding claims, wherein two superimposed layers (26, 28) having equal or different thicknesses (a, i) are formed by the slit (21).

5. A resilient support according to any one of the preceding claims, wherein the slit is provided approximately in the neutral zone of the resilient support.

6. A resilient support according to any one of the preceding claims, wherein the heel part (20) is curved in the opposite direction with respect to the foot-side and lower leg-side end portions (14, 16).

7. A resilient support according to claims 3 to 6, wherein the slit ends (22, 24) are interwoven with a Kevlar thread (34).

8. A resilient support according to any one of the preceding claims, wherein a separating film for separating the layers (26, 28) is provided in the slit (21).

9. A resilient support according to any one of the preceding claims, wherein the resilient support (12) is a leaf spring of fiber-reinforced plastic material.

10. A resilient support according to claim 9, wherein the leaf spring is made either of carbon fiber-reinforced plastic material, glass fiber-reinforced plastic material or another composite material.

11. A resilient support according to any one of the preceding claims, wherein an insert member (36) is introduced into the slit (21).

12. A resilient support according to claim 11, wherein the material of the insert member (36) is a plastic material, especially an elastomer.

13. A resilient support according to claim 11 or 12, wherein the insert member (36) is adhesively joined to the resilient support (12) at an inner surface (42) and is free at an outer surface (44).

14. A resilient support according to claim 11 or 12, wherein the insert member (36) includes insert portions having a different Shore hardness.

15. A resilient support according to any one of the preceding claims, wherein at least one protective member (46) is arranged at the surface of the resilient support (12).

16. A resilient support according to claim 15, wherein the protective member (46) is adhesively joined to the surface of the resilient support (12).

17. A resilient support according to claim 15 or 16, wherein the protective member (46) is arranged at the inner surface (50) and the outer surface (52) of the resilient support (12) in the area of the lower leg-side resilient support portion (54) and the heel part (20).

## Revendications

1. Ressort d'appui, pour une orthèse de jambe, servant à relier de manière articulée un manchon de jambe et un manchon de pied, comprenant une section d'extrémité côté jambe et une section d'extrémité côté pied (14, 16), lesquelles sont reliées entre elles par l'intermédiaire d'une partie talon (20), sachant que les constantes de rappel dudit ressort d'appui sont différentes en fonction de l'angle de rotation (α) du pied, **caractérisé en ce qu'**au moins une entaille (21) est prévue dans la zone du talon.

2. Ressort d'appui selon la revendication 1, sachant que l'entaille (21) est fermée en direction de la section d'extrémité côté jambe et de la section d'extrémité côté pied (14, 16).

3. Ressort d'appui selon la revendication 2, sachant que l'entaille (21) comporte une extrémité d'entaille côté jambe et une extrémité d'entaille côté pied (22, 24), lesquelles sont respectivement tissées ou collées.

4. Ressort d'appui selon l'une quelconque des revendications précédentes, sachant que ressortent à travers l'entaille (21) deux couches (26, 28) superposées, qui présentent des épaisseurs identiques ou différentes (a, i).

5. Ressort d'appui selon l'une quelconque des revendications précédentes, sachant que l'entaille se trouve approximativement dans la zone neutre du ressort d'appui.

6. Ressort d'appui selon l'une quelconque des revendications précédentes, sachant que la partie talon (20) est courbée en sens inverse par rapport à la section d'extrémité côté pied et à la section d'extrémité côté jambe (14, 16).

7. Ressort d'appui selon les revendications 3 à 6, sachant que les extrémités d'entaille (22, 24) sont tissées avec un fil de kevlar (34).

8. Ressort d'appui selon l'une quelconque des revendications précédentes, sachant qu'un film de séparation se trouve dans l'entaille (21), lequel sépare les couches (26, 28).

9. Ressort d'appui selon l'une quelconque des revendications précédentes, sachant que le ressort d'appui (12) est un ressort à lames constitué d'une matière plastique renforcée par des fibres.

10. Ressort d'appui selon la revendication 9, sachant que le ressort à lames est constitué soit d'une matière plastique renforcée par des fibres de carbone, d'une matière plastique renforcée par des fibres de verre ou d'un autre matériau composite.

11. Ressort d'appui selon l'une quelconque des revendications précédentes, sachant qu'un élément d'insertion (36) est installé dans l'entaille (21).

12. Ressort d'appui selon la revendication 11, sachant que le matériau de l'élément d'insertion (36) est une matière plastique, en particulier un élastomère.

13. Ressort d'appui selon la revendication 11 ou 12, sachant que l'élément d'insertion (36) est relié par liaison de matière, au niveau d'une surface intérieure (42), au ressort d'appui (12) et est libre au niveau d'une surface extérieure (44).

14. Ressort d'appui selon la revendication 11 ou 12, sachant que l'élément d'insertion (36) comporte des sections d'insertion présentant différentes duretés Shore.

15. Ressort d'appui selon l'une quelconque des revendications précédentes, sachant qu'au moins un élément de protection (46) est disposé au niveau de la surface du ressort d'appui (12).

16. Ressort d'appui selon la revendication 15, sachant que l'élément de protection (46) est relié par liaison de matière à la surface du ressort d'appui (12).

17. Ressort d'appui selon la revendication 15 ou 16, sachant que l'élément de protection (46) est disposé, au niveau de la surface intérieure (50) et de la surface extérieure (52) du ressort d'appui (12), dans la zone de la section de ressort d'appui (54) côté jambe et dans la partie talon (20).
